(19)

Europäisches
Patentamt
European
Patent Office
Office européen
des brevets

(11)  **EP 1 970 393 A1**

(12)   **EUROPEAN PATENT APPLICATION**

(43) Date of publication:
**17.09.2008  Bulletin 2008/38**

(51) Int Cl.:
*C08G 18/76* *(2006.01)*     *C08G 18/78* *(2006.01)*
*C08G 18/79* *(2006.01)*

(21) Application number: **08004049.6**

(22) Date of filing: **05.03.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **14.03.2007  US 717907**

(71) Applicant: **Bayer MaterialScience LLC
Pittsburgh, PA 15205 (US)**

(72) Inventors:
 • **Garrett, James
  Gordonsville,VA 22942 (US)**
 • **Roesler, Richard R.
  Wexford, PA 15090-7583 (US)**
 • **Kaushiva, Bryan D.
  Baltimore, MD 21224 (US)**

 • **Clatty, Jan L.
  Moon Township, PA 15108-2823 (US)**
 • **Starcher, Rick V.
  Monaca, PA 15061-2637 (US)**
 • **Sebroski, John R.
  Monaca, PA 15061 (US)**
 • **Miller, Jason W.
  Valley Grove, WV 26060 (US)**
 • **Blaszkiewicz, Michael A.
  Gibsonia, PA 15044-8265 (US)**
 • **Masciantonio, Michael A.
  Aliquippa, PA 15001-1474 (US)**
 • **Wellman, Michael T.
  Moundsville, WV 26041-1738 (US)**

(74) Representative: **Weismantel, Lothar
  Bayer MaterialScience AG,
  Law & Patents
  Patents and Licensing
  51368 Leverkusen (DE)**

(54)   **Trimer and allophanate modified isocyanates, a process for their production, foams
     comprising these modified isocyanates, and a process for the production of these foams**

(57)    This invention relates to stable liquid, trimer and allophanate-modified isocyanates, a process for the production
of these isocyanates, foams prepared from these isocyanates and a process for the production of these foams.

**EP 1 970 393 A1**

**Description**

BACKGROUND OF THE INVENTION

This invention relates to trimer and allophanate modified isocyanates, a process for their production, foams comprising these trimer and allophanate modified isocyanates and a process for the production of the foams.

The trimerization of aromatic isocyanates to form polyisocyanurates is well known in the art. U.S. Patents 4,743,627 and 4,382,125 both describe the partial trimerization of polymethylene polyphenylene polyisocyanate (p-MDI), having an average functionality of > 2.2, to give stable liquid products having relatively high viscosity at 25°C (i.e., 2000-100,000 mPa·s). Trimerization of monomeric MDI which is partially converted to carbodiimide/uretonimine, to give stable liquid polyisocyanurate compositions is described in U.S. Patent 4,284,730.

U.S. Patent 5,124,370 describes liquid polyisocyanate mixtures containing isocyanurate groups and having an NCO content of 15 to 30%. These mixtures are obtained by partial trimerization of the isocyanate groups of polyisocyanate mixtures of the diphenylmethane series containing 80 to 100% by weight diisocyanate diphenylmethane isomers and 0 to 20% by weight higher ring compounds of the diphenylmethane series, in which the monomer consists of 20 to 60% by weight of the 2,4'-isomer, 0 to 8% by weight of the 2,2'-isomer, and 40 to 80% by weight of the 4,4'-isomer.

U.S. Patent 5,905,151 describes trimer catalyst systems for aliphatic and aromatic isocyanates. These trimer catalyst systems comprise (A) a lithium compound selected from the group consisting of: (i) lithium salts of aliphatic or aromatic monocarboxylic or dicarboxylic acids, (ii) lithium salts of hydroxyl group containing compounds having from 1 to 3 hydroxyl groups per compound, wherein the hydroxyl groups are directly attached to an aromatic ring, and (iii) lithium hydroxide; and (B) an organic compound containing at least one hydroxyl group. The resultant partially trimerized isocyanates can additionally contain a significant amount of urethane groups.

Another trimer catalyst system for both aliphatic and aromatic isocyanates is disclosed in U.S. Patents 5,955,609 and 6,127,308. This system comprises (A) one or more compounds selected from the group consisting of (i) lithium salts of aliphatic or aromatic carboxylic acids, (ii) lithium salts of hydroxyl group containing compounds where the hydroxyl group is attached to an aromatic ring and (iii) lithium hydroxide; (B) an allophanate catalyst; and (C) an organic compound containing at least one hydroxyl group. The partially trimerized isocyanates may also contain allophanate groups and/or urethane groups.

U.S. Patents 6,028,158 and 6,063,891 disclose freeze-stable, allophanate-modified toluene diisocyanurates having an NCO group content of about 15 to about 42%. These freeze-stable compositions are prepared by reacting A) toluene diisocyanate, and B) an organic compound containing at least one hydroxyl group, in the presence of a catalytic amount of C) at least one allophanate-trimer catalyst, or an allophanate-trimer catalyst system. These compositions contain both isocyanurate groups and allophanate groups. Blends of these allophanate-modified toluene diisocyanurates with polymethylene poly(phenylisocyanates) are disclosed; as well as urethane prepolymers of these allophanate-modified toluene diisocyanurates, and blends of these with PMDI.

U.S. Patents 4,255,659, 4,518,761, 4,772,639 and 5,798,431 all describe trimerization products of isocyanate components and processes for their production. None of these disclose liquid trimer products prepared exclusively from di- or polyisocyanates of the diphenylmethane series. U.S. Patents 4,255,659 and 4,518,761 specifically require two isocyanate components which have different reactivities with respect to trimerization. U.S. Patents 4,772,639 and 5,798,431 disclose polyisocyanates containing isocyanurate groups or trimers in which at least one of the isocyanate components has aliphatically or cycloaliphatically bound isocyanate groups.

U.S. Patent 5,102,918 describes a process for producing a modified organic polyisocyanate having an isocyanurate ring. This process comprises adding a trimerization catalyst, an organic phosphite ester and a surfactant (and optionally a ferrocene compound) to an organic polyisocyanate and/or a partially urethanized organic polyiscyanate to form isocyanurate groups of not more than 20% of the total of isocyanate groups. A stopper may be added. Suitable organic polyisocyanates include both TDI and MDI.

U.S. Patents 4,326,043, 4,359,541 and 4,359,550 each describes dispersible polyisocyanurate polymers. Suitable isocyanates are disclosed broadly, including mixtures of TDI, MDI and PMDI. This reference also discloses that the isocyanate can be converted to a trimer in a solvent which is a solid, and then dispersed in a polyol. Examples 48-84 of the '043 patent disclose the dispersed trimer solid containing catalysts, surfactants, etc., is reacted with the isocyanate blend of TDI/MDI (80:20) to form a foam.

Stable solutions of trimerized isocyanate prepolymers in monomeric polyisocyanates are described by the 4,552,902 patent. First an NCO-terminated prepolymer is made, then a cotrimer is formed by trimerizing the NCO-terminated prepolymer with MDI or PMDI. The cotrimer is reacted with an excess of a low equivalent weight polyol to form another isocyanate-terminated prepolymer. TDI can be used to form the first NCO-terminated prepolymer. The examples all use TDI and MDI, and various polyols to form prepolymers. It is expressly stated (column 5, lines 50-55), that the diols are required to make liquid products. Allophanate formation may result from trimerizing the mixture of the second isocyanate with the first NCO-terminated prepolymer.

GB 1,337,659 describes a polyisocyanate solution which comprises a solution of at least one polyisocyanate containing at least one isocyanuric acid ring dissolved in a monomeric polyisocyanate which is free from isocyanurate groups. These are a TDI trimer mixed with a TDI prepolymer, not mixed trimers. Each of the final isocyanate products in these working examples contains some urethane and/or allophanate groups, and a relatively small quantity of trimer groups.

Allophanate-modified isocyanates based on diphenylmethane diisocyanate which are storage stable liquids are known and described in, for example, U.S. Patents 5,319,053, 5,319,054, 5,663,272, 5,783,652, 6,482,913, 6,887,399 and 6,991,746.

U.S. 5,663,272 is specific to prepolymers of allophanate-modified MDI. These prepolymers are prepared by reacting an organic material containing OH groups with a monoisocyanate, reacting this product with a mixture of MDI isocmers, and reacting this product with a compound containing isocyanate-reactive groups to form the stable, liquid allophanate-modified MDI prepoymer.

U.S. Patent 5,783,652 discloses mixtures of urethane prepolymers of allophanate-modified diphenylmethane diisocyanates with epoxides. The addition of the epoxide increases the reactivity of the polymers.

Liquid MDI adducts having improved freeze stability are disclosed in U.S. Patent 6,482,913. These liquid MDI compositions comprise an allophanate-modified MDI, a low molecular weight branched aliphatic dihydroxy compound and an epoxide functional compound.

Polymeric allophanates of diphenylmethane diisocyanates, the prepolymers thereof and processes for their preparation are described in U.S. Patents 6,887,399 and 6,991,746. These are higher functionality allophanate products (i.e. polymeric allophanates) and prepolymers of these higher functionality allophanate products. Conventional alcohol or diol based allophanate modified MDI is further converted, either partially or completely, to higher functionality allophanate products.

U.S. Published Patent Applications 2005/0101754 A1 and 2006/0073321 A1 describe stable liquid, allophanate-modified, partially trimerized diphenylmethane diisocyanates, prepolymers thereof, processes for their preparation, foams prepared from these stable liquid products and processes for the production of foams.

Advantages of the presently claimed storage stable, allophanate-modified diphenylmethane diisocyanate trimers include the fact that these products do not contain a significant quantity of allophanates of 2,4'-diphenylmethane diisocyanate due to the narrow isomer distribution range of the MDI that is allophanatized. It has been found that products prepared by first forming the allophanate of high 4,4'-MDI, blending this with an isomeric mixture of MDI and then trimerizing the blend offers the advantages/unexpected results of being storage stable despite having large amounts of trimerized 4,4'-MDI present and low levels of the 2,4'-MDI in the mixture.

## SUMMARY OF THE INVENTION

This invention relates to stable liquid, allophanate-modified, partially trimerized diphenylmethane diisocyanates having NCO group contents of 10 to 30% by weight. These comprise the trimerization product of:

(A) from 75 to 90% by weight, based on 100% by weight of (A) and (B), of an allophanate-modified diphenylmethane diisocyanate component having an NCO group content of 23% to 29%, and which comprises the reaction product of:

(1) diphenylmethane diisocyanate which comprises from 85% to 100% by weight of the 4,4'-isomer, from 0 to 9% by weight of the 2,4'-isomer and from 0 to 6% by weight of the 2,2'-isomer, with the sum of the %'s by weight of the 4,4'-isomer, the 2,4'-isomer and the 2,2'-isomer totaling 100% by weight of the diphenylmethane diisocyanate component;
with
(2) an organic compound having from 1 to 36 carbon atoms and containing at least one hydroxyl group (preferably an aromatic alcohol having from 6 to 18 carbon atoms or more preferably an aliphatic alcohol having from 1 to 36 carbon atoms);
in the presence of
(3) an allophanate catalyst;

and
(B) from 10 to 25% by weight, based on 100% by weight of (A) and (B), of a diphenylmethane diisocyanate component having an NCO group content of about 33 to about 34% by weight, and containing from 31% to 55% by weight of the 4,4'-isomer, from 45% to 65% by weight of the 2,4'-isomer and from 0 to 4% by weight of the 2,2'-isomer, with the sum of the %'s by weight of the 4,4'-isomer, the 2,4'-isomer and the 2,2'-isomer totaling 100% by weight of the diphenylmethane diisocyanate component;
in the presence of

(C) at least one trimerization catalyst; with the proviso that the clarity of the allophanate-modified, partially trimerized diphenylmethane diisocyanate product after 2 days is $\geq 0.8$, in which:

$$0.8 \leq ((17.19 \times WF_A) + (0.94 \times WF_B) - (0.54 \times WF_A \times \%NCO_P) -$$

$$(0.70 \times WF_A \times \%NCO_A) + (0.02 \times WF_A \times \%NCO_P \times \%NCO_A))$$

wherein:

$WF_A$: represents the weight fraction of the allophanate-modified diphenylmethane diisocyanate component (A);
$WF_B$: represents the weight fraction of the diphenylmethane diisocyanate component (B);
$NCO_A$: represents the NCO group content of the allophanate-modified diphenylmethane diisocyanate component (A);
and
$NCO_p$: represents the NCO group content of the allophanate-modified, partially trimerized diphenylmethane diisocyanate product which ranges from 10% to 30%.

The process of preparing these stable liquid, allophanate-modified, partially trimerized diphenylmethane diisocyanates comprises:

(I) trimerizing a blend comprising:

(A) from 75 to 90% by weight, based on 100% by weight of (A) and (B), of an allophanate-modified diphenylmethane diisocyanate component having an NCO group content of 23% to 29%, and which is the reaction product of:

(1) diphenylmethane diisocyanate which comprises from 85% to 100% by weight of the 4,4'-isomer, from 0 to 9% by weight of the 2,4'-isomer and from 0 to 6% by weight of the 2,2'-isomer, with the sum of the %'s by weight of the 4,4'-isomer, the 2,4'-isomer and the 2,2'-isomer totaling 100% by weight of the diphenylmethane diisocyanate component;
with
(2) an organic compound having from 1 to 36 carbon atoms and containing at least one hydroxyl group (preferably an aromatic alcohol having from 6 to 18 carbon atoms, or more preferably an aliphatic alcohol having from 1 to 36 carbon atoms);
in the presence of
(3) an allophanate catalyst;

with
(B) from 10 to 25% by weight, based on 100% by weight of (A) and (B), of a diphenylmethane diisocyanate component having an NCO group content of about 33 to about 34% by weight, and containing from 31 % to 55% by weight of the 4,4'-isomer, from 45% to 65% by weight of the 2,4'-isomer and from 0 to 4% by weight of the 2,2'-isomer, with the sum of the %'s by weight of the 4,4'-isomer, the 2,4'-isomer and the 2,2'-isomer totaling 100% by weight of the diphenylmethane diisocyanate component;
in the presence of:
(C) at least one trimerization catalyst;

and
(II) adding a suitable catalyst stopper once the desired NCO group content is attained;

with the proviso that the clarity of the allophanate-modified, partially trimerized diphenylmethane diisocyanate product after 2 days is $\geq 0.8$, and in which:

$$0.8 \leq ((17.19 \times WF_A) + (0.94 \times WF_B) - (0.54 \times WF_A \times \%NCO_P) -$$

$$(0.70 \times WF_A \times \%NCO_A) + (0.02 \times WF_A \times \%NCO_P \times \%NCO_A))$$

wherein WF$_A$, WF$_B$, NCO$_A$ and NCOp are defined as above.

The present invention also relates to a process for the preparation of a polyurethane foams comprising reacting a polyisocyanate with an isocyanate-reactive component in the presence of one or more catalysts, one or more blowing agents, and at least one surfactant, in which the polyisocyanate component comprises the stable liquid, allophanate-modified, partially trimerized diphenylmethane diisocyanate described above. The foams produced by this process are also part of the present invention.

BRIEF DESCRIPTION OF THE DRAWINGS

Figure 1 is a graph illustrating the product clarity after 2 days for allophanate-modified, partially trimerized diphenylmethane diisocyanates in which the allophanate component has an NCO group content of from 23% to 29% by weight, and the product has an NCO group content of from about 20% to about 29% by weight. These products comprised 90% by weight of allophanate modified diphenylmethane diisocyanate and 10% by weight of a diphenylmethane diisocyanate having a high 2,4'-isomer content.

Figure 2 is a graph illustrating the product clarity after 2 days for allophanate-modified, partially trimerized diphenylmethane diisocyanates in which the allophanate component has an NCO group content of from 23% to 29% by weight, and the product has an NCO group content of from about 20% to about 29% by weight. These products comprised 75% by weight of allophanate modified diphenylmethane diisocyanate and 25% by weight of a diphenylmethane diisocyanate having a high 2,4'-isomer content.

DETAILED DESCRIPTION OF THE INVENTION

In accordance with the present invention, the term "stable liquid " means that the allophanate-modified, partially trimerized diphenylmethane diisocyanate product has up to a 1 % absolute change in the % NCO group content and up to a 10% change in the viscosity when stored at 25°C for 2 days, preferably at least 7 days, more preferably at least 1 month and most preferably at least 3 months.

The term "clarity" as used herein with respect to the allophanate-modified, partially trimerized diphenylmethane diisocyanate of the present invention refers to the clearness or transparency of these products after 2 days storage at room temperature. More specifically, the products of the invention are characterized by a clarity of at least 0.8 on a scale of 0 to 1; in which 0 represents a turbid or opaque product, 0.5 represents a hazy product, 0.8 represents a slightly hazy or partially clear or transparent product, and 1 represents a completely transparent or clear product. As set for the above, the allophanate-modified, partially trimerized diphenylmethane diisocyanate of the invention have a clarity of $\geq$ 0.8 in which:

$$0.8 \leq ((17.19 \times WF_A) + (0.94 \times WF_B) - (0.54 \times WF_A \times \%NCO_P) -$$
$$(0.70 \times WF_A \times \%NCO_A) + (0.02 \times WF_A \times \%NCO_P \times \%NCO_A))$$

wherein: WF$_A$, WF$_B$, NCO$_A$ and NCO$_P$ are defined as above.

The statistical significance of the above equation is 99.9%.

In accordance with the present invention, stable liquid allophanate-modified, partially trimerized diphenylmethane diisocyanates having a clarity as low as 0.8 which is determined by the above formula are also suitable/useful herein.

In the stable liquid, allophanate-modified, partially trimerized diphenylmethane diisocyanates of the present invention and the process(es) for making them, the following components are, generally speaking, suitable.

The allophanate-modified diphenylmethane diisocyanate component (A) herein typically has an NCO group content of 23 to 29% by weight, preferably 25 to 29% by weight and more preferably 27 to 29% by weight. It is also typically present in an amount ranging from 75 to 90% by weight, preferably from 78 to 88% by weight and more preferably 80 to 85% by weight, based on 100% by weight of components (A) and (B).

Suitable allophanate-modified diphenylmethane diisocyanates comprise the reaction product of (A)(1) a diphenylmethane diisocyanate component having the specified isomer distribution and (A)(2) an organic compound having from 1 to 36 carbon atoms and containing at least one hydroxyl group.

Suitable diphenylmethane diisocyanates to be used as component (A)(1) comprise (i) from 85 to 100% by weight of 4,4'-diphenylmethane diisocyanate, (ii) from 0 to 9% by weight of 2,4'-diphenylmethane diisocyanate, and (iii) from 0 to 6% by weight of 2,2'-diphenylmethane diisocyanate, with the %'s by weight of (A)(1)(i), (A)(1)(ii) and (A)(1)(iii) totaling 100% by weight of (A)(1). The % by weight of (A)(1)(i) the 4,4'-isomer of diphenylmethane diisocyanate is typically at least about 85%, and preferably at least about 90%, more preferably at least about 94%. The % by weight of (A)(1) (i) the 4,4'-isomer generally is about 100% or less, and preferably about 98% or less. The diphenylmethane diisocyanate component (A)(1) may have (i) a 4,4'-isomer content ranging between any of these upper and lower values, inclusive, e.g., from 85 to 100%, preferably from 90 to 98% and more preferably from 94 to 98%. The % by weight of the (ii) 2,4'-isomer of diphenylmethane diisocyanate is typically about 0% or more, and preferably about 0.5% or more. The % by weight of (ii) the 2,4'-isomer generally is about 9% or less, and preferably about 4% or less. The diphenylmethane diisocyanate component (A)(1) may have (ii) a 2,4'-isomer content ranging between any of these upper and lower values, inclusive, e.g., from about 0 to about 9%, and preferably from about 0.5 to about 4%. The % by weight of (iii) the 2,2'-isomer of diphenylmethane diisocyanate is typically at least about 0%, and preferably at least about 0.05%. The % by weight of (iii) the 2,2'-isomer generally is about 6% or less, and preferably of about 0.5% or less. The diphenylmethane diisocyanate component (A)(1) may have (iii) a 2,2'-isomer content ranging between any of these upper and lower values, inclusive, e.g., from 0 to 6% ,and preferably from 0 to 0.5%. The sum of the amounts of the isomers (i), (ii) and (iii) always totals 100% by weight of (A)(1) the diphenylmethane diisocyanate.

Some examples of preferred isocyanates include isomeric mixtures of diphenylmethane diisocyanate (A)(1) containing from about 97 to 99.5% of the 4,4'-isomer, from about 0 to about 0.5% of the 2,4'-isomer and from about 0 to about 0.1 % of the 2,2'-isomer (with the %'s by weight of the isomers totaling 100%).

Organic compounds having from 1 to 36 carbon atoms and which contain at least one hydroxyl group are suitable to be used as component (A)(2) in accordance with the present invention. Suitable compounds for component (A)(2) of the present invention also typically include aliphatic alcohols containing from 1 to 36 carbon atoms and preferably from 2 to 16 carbon atoms and aromatic alcohols containing from 6 to 18 carbon atoms and preferably from 6 to 12 carbon atoms. These organic compounds typically contain at least about 1 hydroxyl group. Also, these organic compounds also typically contain no more than about 4 hydroxyl groups, preferably no more than about 3 hydroxyl groups, and most preferably no more than about 2 hydroxyl groups. The organic compound may contain any number of hydroxyl groups ranging between any combination of these upper and lower values, inclusive, e.g., from about 1 to about 4, preferably from about 1 to about 3, and most preferably from about 1 to about 2.

Some illustrative but non-limiting examples of aliphatic alcohols to be used as the organic compounds for component (A)(2) herein include, conventional aliphatic alcohols, cycloaliphatic alcohols, aliphatic alcohols containing aromatic groups, and aliphatic alcohols containing groups that do not react with isocyanates such as, for example, ether groups and halogens such as bromine and chlorine. More specific examples of aliphatic alcohols suitable for the present invention include compounds such as methanol, ethanol, 1,2-ethanediol, 2-methoxyethanol, 2-bromoethanol, 1-propanol, 2-propanol, 1-butanol, isobutyl alcohol, 2-butanol, n-amyl alcohol, sec-amyl alcohol, tert-amyl alcohol, 1-ethyl-1-propanol, n-hexanol and isomers thereof, n-octyl alcohol, 2-octyl alcohol, 2-ethyl-1-hexanol, n-decyl alcohol, n-dodecyl alcohol, neopentylglycol, n-tetradecyl alcohol, n-hexadecyl alcohol, n-octadecyl alcohol, 1,3-propanediol, 1,4-butanediol, 1,3-butanediol, 2,3-butanediol, 3-methyl-2-butanol, 3,3-dimethyl-1-butanol, 2-ethyl-1,3-hexanediol, glycerol, 1,2,4-butanetriol, pentaerythritol, diethylene glycol, dipropylene glycol, diethylene glycol, triethylene glycol, etc. It is more preferred for these organic compounds to contain from 1 to 2 hydroxyl groups, such as a monoalcohol or a diol, and have a molecular weight of from 60 to about 200. Examples include 1-propanol, 2-propanol, 1-butanol, 2-butanol, n-amyl alcohol, 1-methylbutyl alcohol, 1-ethyl-1-propanol, n-octyl alcohol, 2-octyl alcohol, 2-ethyl-1-hexanol, neopentyl-glycol, 1,2-propanediol, 1,3-propanediol, 1,4-butanediol, 1,3-butanediol, 2,3-butanediol, 2-ethyl-1,3-hexanediol, diethylene glycol, triethylene glycol, dipropylene glycol, tripropylene glycol, etc. Preferred compounds are isomeric alcohols having between 2 and 16 carbon atoms such as, for example, the isomeric butanols, and the isomeric propanols. Most preferred are 2-propanol and isobutyl alcohol.

Suitable aromatic alcohols to be used as component (A)(2) in the present invention include those alcohols having the alcoholic hydroxyl group attached directly to the aromatic ring. Some specific examples of such aromatic alcohols for (A)(2) include, for example, phenol, m-cresol, 1-naphthol, 2-naphthol, o-chlorophenol, p-bromophenol, m-nitorphenol, o-fluorophenol, etc.

In accordance with the present invention, (A)(2) the organic compound containing at least one hydroxyl group is typically present in a quantity such that there are from about 0.01 to about 0.25 equivalent hydroxyl group per equivalent of diphenylmethane diisocyanate (A)(1) present. It is preferred that there are from about 0.01 to about 0.2, more preferably about 0.03 to about 0.18, most preferably about 0.05 to about 0.15 and most particularly preferably about 0.07 to about 0.12 equivalent hydroxyl group per equivalent of diphenylmethane diisocyanate (A)(1) present. Also, there should be at least about 50%, preferably at least about 70%, more preferably at least about 80% of the equivalents of hydroxyl groups present in (A)(2) the organic compound which contains hydroxyl groups which are converted from urethane groups to allophanate groups in the final product. Most preferably, at least about 90% (and most particularly preferably 95%) of the equivalents of hydroxyl groups present in (A)(2) the organic compound which contains hydroxyl groups are converted from urethane groups to allophanate groups.

The reaction between components (A)(1) and (A)(2) typically occurs in the presence of a suitable (A)(3) a suitable allophanate catalyst. General process conditions for the allophanate formation are known and described in, for example, U.S. Patents 5,319,053, 5,319,054 and 5,440,003, the disclosures of which are hereby incorporated by reference. Suitable allophanate catalysts to be used herein as (A)(3) include, for example, metal carboxylates and metal acetylacetonates. Some specific examples of suitable allophanate catalysts are zinc octoate, tin-2-ethylhexanoate, zinc acetylacetonate (Zn AcAc), zinc-2-ethylhexanoate, cobalt linoresinate, lead naphthenate, lead 2-ethylhexanoate, lead linoresinate, cobalt 2-ethylhexanoate, cobalt naphthenate, etc. Preferred allophanate catalysts are zinc octoate, tin octoate, zinc-2-ethylhexanoate, tin-2-ethylhexanoate, and zinc acetylacetonate.

Allophanate-modified diphenylmethane diisocyanates are known and described in, for example, U.S. Patents 5,319,053, 5,319,054 and 5,440,003, the disclosures of which are hereby incorporated by reference. In fact, there are several allophanate-modified diphenylmethane diisocyanates which are commercially available from Bayer MaterialScience LLC. These commercially available allophanate-modified diphenylmethane diisocyanates may be suitable to be used as component (A) in the novel partially trimerized, allophanate-modified diphenylmethane diisocyanates of the present invention.

It is well known that such commercially available allophanate-modified diphenylmethane diisocyanates typically add a catalyst stopper at the desired end-point (i.e. NCO group content) of the allophanate-reaction to yield storage-stable liquid allophanate-modified products. See U.S. Patents 5,319,053, 5,319,054 and 5,440,003. Suitable catalyst stoppers include, for example, acidic catalyst stoppers such as, for example, anhydrous hydrochloric acid, sulfuric acid, bis(2-ethylhexyl) hydrogen phosphate, benzoyl chloride, Lewis Acids and the like. Preferred catalyst stoppers are benzoyl chloride and bis(2-ethylhexyl) hydrogen phosphate. The quantity of stopper used is the amount needed to deactivate the catalysts. This quantity will vary depending on the catalyst and stopper used. In general, however, between 30 and 150% by wt. (preferably 50 to 100% by wt.) of stopper relative to the weight of the catalyst is typically needed, but more may be used.

If a commercially available allophanate-modified diphenylmethane diisocyanate is used as component (A) in the present invention, it may be necessary to adjust the quantity of (C) the trimerization catalyst used due to the presence of the catalyst stopper in these products. Due to variations in the strength of trimerization catalysts, the necessary quantity of these trimerization catalysts (C) to attain a reasonable and/or the desired reaction rate may vary widely.

Suitable diphenylmethane diisocyanates to be used as component (B) in the present invention include those having NCO group contents of about 33 to about 34% by weight. This component is typically present in an amount ranging from 10 to 25% by weight, preferably from 12 to 22% by weight, and more preferably 15 to 20% by weight, based on 100% by weight of (A) and (B).

The suitable diphenylmethane diisocyanates comprise (i) from about 31 to about 55% by weight of the 4,4'-isomer, (ii) from about 45 to about 65% by weight of the 2,4'-isomer and (iii) from about 0 to about 4% by weight of the 2,2'-isomer, with the %'s by weight of (B)(i), (B)(ii) and (B)(iii) totaling 100% by weight of (B). The % by weight of (B)(i) the 4,4'-isomer of diphenylmethane diisocyanate is typically at least about 31 %, and preferably at least about 40%. The % by weight of (B)(i) the 4,4'-isomer generally is about 55% or less, and preferably about 50% or less. The diphenylmethane diisocyanate component (B) may have (i) a 4,4'-isomer content ranging between any of these upper and lower values, inclusive, e.g., from 31 to 55%, and preferably from 40 to 50%. The % by weight of the (ii) 2,4'-isomer of diphenylmethane diisocyanate is typically about 45% or more, and preferably about 50% or more. The % by weight of (ii) the 2,4'-isomer generally is about 65% or less, and preferably about 60% or less. The diphenylmethane diisocyanate component (B) may have (ii) a 2,4'-isomer content ranging between any of these upper and lower values, inclusive, e.g., from about 45 to about 65%, and preferably from about 50 to about 60%. The % by weight of (iii) the 2,2'-isomer of diphenylmethane diisocyanate is typically at least about 0%, and preferably at least about 1%. The % by weight of (iii) the 2,2'-isomer generally is about 4% or less, and preferably of about 2% or less. The diphenylmethane diisocyanate component (A)(1) may have (iii) a 2,2'-isomer content ranging between any of these upper and lower values, inclusive, e.g., from 0 to 4%, and preferably from 1 to 2%. The sum of the amounts of the isomers (i), (ii) and (iii) always totals 100% by weight of (B) the diphenylmethane diisocyanate.

A particular example of a diphenylmethane diisocyanate to be used as component (B) herein contains (i) from about 40 to 50% of the 4,4'-isomer, (ii) from about 50 to about 60% of the 2,4'-isomer and (iii) from about 0 to about 2% of the 2,2'-isomer (with the %'s by weight of the isomers totaling 100% by weight of (B)).

In accordance with the present invention, components (A) and (B) are blended in the relative quantities as set forth above, and the blend is trimerized in the presence of (C) a suitable trimerization catalyst. Examples of suitable trimer catalysts include alkali carboxylates as described in U.S. Patent 4,604,418, the disclosure of which is herein incorporated by reference; basic alkali metal salts complexed with acyclic organic compounds as described in U.S. Patent 4,379,905, the disclosure of which is herein incorporated by reference; basic alkali metal salts complexed with crown ethers as described in U.S. Patent 4,487,928, the disclosure of which is herein incorporated by reference; combinations of tertiary amines with specific quaternary ammonium salts as described in U.S. Patent 3,954,684, the disclosure of which is herein incorporated by reference; and alkali metals salts or quaternary ammonium salts of carboxylic acids which correspond to one of several different structures as described in U.S. Patents 4,632,785 and 4,540,781, the disclosures of which are herein incorporated by reference; various lithium salts of monocarboxylic acids, lithium salts of hydroxyl group containing compounds and lithium hydroxide, in combination with an organic compound having at least one hydroxyl group as described in, for example, U.S. Patent 5,905,151; etc. Other known trimer catalysts include, for example, catalyst which inherently form both trimer groups and allophanate groups. Among these trimer catalysts are included the Mannich bases such as, for example, dimethylaminophenol and 2,4,6-bis(dimethylaminomethyl)-phenol; and metal salts of carboxylic acids such as, for example, lead octanoate and potassium acetate.

Generally speaking, the trimerization process occurs at temperatures of from about 70°C to about 120°C for time periods of about 1 to about 4 hours. Trimerization processes as described in, for example, U.S. Patent 5,124,370, U.S. Patent 5,905,151 and U.S. Patent 6,127,308, the disclosures of which are hereby incorporated by reference, are also suitable herein.

In accordance with the process of the present invention, a catalyst stopper is added to the reaction process once the desired NCO group content of the stable liquid, partially trimerized, allophanate-modified diphenylmethane diisocyanate herein. These products typically have NCO group contents ranging from 10 to 30% by weight, preferably from 15 to 30% by weight and more preferably from 20 to 30% by weight.

Reference will now be made to Figure 1. Figure 1 is a graph which illustrates the product clarity after 2 days for allophanate-modified, partially trimerized diphenylmethane diisocyanates. These allophanate-modified, partially trimerized diphenylmethane diisocyanates comprise (A) 90% by weight of an allophanate-modified diphenylmethane diisocyanate which has an NCO group content of from about 23% to about 29%, and (B) 10% by weight of a diphenylmethane diisocyanate which has a high 2,4'-MDI isomer content. More specifically, this diphenylmethane diisocyanate (B) contains about 42% by weight of the 4,4'-isomer, about 57% by weight of the 2,4'-isomer and less than about 1% by weight of the 2,2'-isomer. Figure 1 shows the product clarity as ranging from 0.0 to 1.0 for the final product (i.e. allophanate-modified, partially trimerized diphenylmethane diisocyanate) over the NCO group content for the product of about 20% to about 29% NCO (x-axis) plotted against an % NCO group content of about 23% to about 29% for component (A) the allophanate-modified diphenylmethane diisocyanate (y-axis).

In accordance with the present invention, a product clarity of:

0.0    represents a turbid or opaque product which is unsuitable for the present invention,
0.5    represents a hazy product which is unsuitable for the present invention,
0.8    represents a very slightly hazy product which is suitable for the present invention, and
1.0    represents a clear product which is suitable for the present invention.

As previously stated, only products having a clarity of 0.8 or greater are suitable or acceptable products in accordance with the present invention. The relationship between product clarity and the ingredient variables (i.e. components (A) and (B) as described herein) is very complicated as can seen by the curvilinear relationship. See Figure 1. Thus, the following equation was used to determine the clarity of the final product as shown in Figure 1 which ranges from 0.0 to 1.0:

$$\text{Clarity}_P = ((17.19 \times WF_A) + (0.94 \times WF_B) - (0.54 \times WF_A \times \%NCO_P) -$$

$$(0.70 \times WF_A \times \%NCO_A) + (0.02 \times WF_A \times \%NCO_P \times$$

$$\%NCO_A))$$

wherein:

| | |
|---|---|
| $\text{Clarity}_P$: | represents the clarity of the final product; |
| $WF_A$: | represents the weight fraction of the allophanate-modified diphenylmethane diisocyanate component (A); |
| $WF_B$: | represents the weight fraction of the diphenylmethane diisocyanate component (B); |
| $NCO_A$: | represents the NCO group content of the allophanate-modified diphenylmethane diisocyanate component (A); and |
| $NCO_P$:represents | the NCO group content of the allophanate-modified, partially trimerized diphenylmethane diisocyanate product which ranges from 10% to 30%. |

Reference will now be made to Figure 2. Figure 2 is a graph which illustrates the product clarity after 2 days for allophanate-modified, partially trimerized diphenylmethane diisocyanates. These allophanate-modified, partially trimerized diphenylmethane diisocyanates comprise (A) 75% by weight of an allophanate-modified diphenylmethane diisocyanate which has an NCO group content of from about 23% to about 29%, and (B) 25% by weight of a diphenylmethane diisocyanate which has a high 2,4'-MDI isomer content. More specifically, this diphenylmethane diisocyanate (B) contains about 42% by weight of the 4,4'-isomer, about 57% by weight of the 2,4'-isomer and less than about 1 % by weight of the 2,2'-isomer. Figure 2 shows the product clarity as ranging from 0.0 to less than 1.0 for the final product (i.e. allophanate-modified, partially trimerized diphenylmethane diisocyanate) over the NCO group content for the product of about 20% to about 29% NCO (x-axis) plotted against an % NCO group content of about 23% to about 29% for component (A) the allophanate-modified diphenylmethane diisocyanate (y-axis). Due to mathematical averaging, products having a clarity of 1.0 do not actually show up on Figure 2, although these were included in the raw data.

In accordance with the present invention, a product clarity of:

0.0   represents a turbid or opaque product which is unsuitable for the present invention,
0.5   represents a hazy product which is unsuitable for the present invention,
0.8   represents a very slightly hazy product which is suitable for the present invention, and
1.0   represents a clear product which is suitable for the present invention.

As previously stated, only products having a clarity of 0.8 or greater are suitable or acceptable products in accordance with the present invention. The relationship between product clarity and the ingredient variables (i.e. components (A) and (B) as described herein) is very complicated as can seen by the curvilinear relationship. See Figure 2. Thus, the following equation was used to determine the clarity of the final product as shown in Figure 2 which ranges from 0.0 to less than 1.0:

$$Clarity_P = ((17.19 \times WF_A) + (0.94 \times WF_B) - (0.54 \times WF_A \times \%NCO_P) - (0.70 \times WF_A \times \%NCO_A) + (0.02 \times WF_A \times \%NCO_P \times \%NCO_A))$$

wherein: $Clarity_P$, $WF_A$, $WF_B$, $NCO_A$ and $NCO_P$ are defined as above.

In the process for the production of foams, the stable liquid, partially trimerized, allophanate-modified diphenylmethane diisocyanates described above are reacted with an isocyanate-reactive component, in the presence of one or more catalysts, one or more blowing agents (preferably water) and at least one surfactant. The isocyanate index for this reaction is typically between 100 and 130, and preferably between 100 and 115.

Suitable isocyanate-reactive components for the foam and process of making the foam include the known polyether polyols, polyester polyols, polycarbonate diols, polyhydric polythioethers, polyacetals, aliphatic thiols, solids containing polyols including those selected from the group consisting of graft polyols, polyisocyanate polyaddition polyols, polymer polyols, PHD polyols and mixtures thereof, etc. It is also possible to use lower molecular weight compounds such as, for example, those amine and hydroxyl functional compounds which are commonly used as starters in the preparation of polyether polyols. These lower molecular weight compounds are sometimes referred to as chain extenders and/or crosslinkers are also suitable for the isocyanate-reactive component herein. The molecular weights (number average) and functionalities of these isocyanate-reactive components can vary widely. For example, compounds having molecular weights as low as 60 and as high as 8,000 and functionalities as low as about 2 and as high as about 8 can be used.

A preferred isocyanate-reactive component for the present invention comprises:

(a) from about 45 to about 75% by weight of at least one aromatic polyester polyol which has a functionality of at least about 2, a molecular weight of from greater than 500 to about 3000, and an OH number of from about 35 to about 2250;
(b) from about 5 to about 15% by weight of at least one organic compound which preferably contains about two hydroxyl groups, has a molecular weight in the range of from about 60 to about 300, and an OH number of about 370 to about 1870; and
(c) from about 20 to about 45% by weight of at least one organic compound which preferably contains from about 3 to about 4 hydroxyl groups, has a molecular weight of about 60 to about 500 and has an OH number of about 330 to about 3750,

with the sum of the %'s by weight of components (a), (b) and c) totaling 100% by weight of the isocyanate-reactive component.

Preferred polyester polyols for (a) have a functionality of 2. These preferred polyester polyols also typically have a molecular weight of greater than about 500, preferably at least about 525 and more preferably at least about 550. The polyester polyols also typically have molecular weights of less than or equal to about 3000, preferably less than or equal to about 2000, and more preferably less than or equal to about 1000. Suitable polyester polyols may also have a molecular weight ranging between any combination of these upper and lower values, inclusive unless expressly stated otherwise. Such polyester polyols may have a molecular weight of greater than 500 to less than or equal to 3000, preferably at least about 525 to less than or equal to about 2000, and more preferably at least about 550 to less than or equal to about 1000.

Suitable organic compounds which contain about 2 hydroxyl groups to be used as component (b) of the isocyanate-reactive component typically have a molecular weight of at least about 60, preferably at least about 100, and more preferably at least about 150. These organic compounds also typically have a molecular weight of less than or equal to 300, preferably less than or equal to 275 and more preferably less than or equal to about 250. Suitable organic compounds may have a molecular weight ranging between any combination of these upper and lower values, inclusive. For example, these organic compounds may have a molecular weight ranging from 60 to 300, preferably from 100 to 275 and more preferably from 150 to 250.

Suitable crosslinking agents to be used as component (c) of the isocyanate-reactive component typically have a molecular weight of at least about 60, preferably at least about 100, and more preferably at least about 150. These organic compounds also typically have a molecular weight of less than or equal to 500. Suitable organic compounds may have a molecular weight ranging between any combination of these upper and lower values, inclusive. For example, these organic compounds may have a molecular weight ranging from 60 to 500, preferably from 100 to 500 and more preferably from 150 to 500.

Suitable catalysts to be used in the preparation of polyurethane foams from the novel stable liquid, allophanate-modiifed, partially trimerized diphenylmethane diisocyanate components herein include any known urethane catalyst. Examples of such catalysts include the known tertiary amine compounds and organometallic compounds. Examples of suitable tertiary amine catalysts include triethylenediamine, N-methylmorpholine, pentamethyl diethylenetriamine, dimethylcyclohexylamine, tetra-methylethylenediamine, 1-methyl-4-dimethylaminoethyl-piperazine, 3-methoxy-N-dimethyl-propylamine, bis[2-dimethylaminoethyl]ether, diazabicyclooctane, N-ethylmorpholine, diethylethanolamine, N-cocomorpholine, N,N-dimethyl-N',N'-dimethylisopropyl-propylene diamine, N,N-diethyl-3-diethyl aminopropylamine and dimethyl-benzyl amine. Examples of suitable organometallic catalysts include organomercury, organolead, organoferric and organotin catalysts, with organotin catalysts being preferred. Suitable organotin catalysts include preferably, tin(II) salts of carboxylic acids, such as tin(II) acetate, tin(ii) octoate, tin(II) ethylhexoate, and tin(II) laurate, as well as tin(IV) compounds, such as dibutyltin dilaurate, dibutyltin dichloride, dibutyltin diacetate, dibutytin maleate, and dioctyltin diacetate. Suitable bismuth compounds include bismuth neodecanoate, bismuth versalate, and various bismuth carboxylates known in the art. Metal salts such as stannous chloride can also function as catalysts for the urethane reaction.

Suitable blowing agents to be used in the preparation of polyurethane foams include but are not limited to compounds such as, for example, water, carbon dioxide, fluorocarbons, chlorofluorocarbons, hydrochlorofluorocarbons, perfluorocarbons, and low boiling hydrocarbons. Some examples of suitable hydrochlorofluorocarbons include compounds such as 1,1-dichloro-1-fluoroethane (HCFC-141b), 1-chloro-1,1-difluoroethane (HCFC-142b), and chlorodifluoro-methane (HCFC-22); of suitable hydrofluorocarbons include compounds such as 1,1,1,3,3-pentafluoropropane (HFC-245fa), 1,1,1,2-tetrafluoro-ethane (HFC-134a), 1,1,1,3,3,3-hexafluoropropane (HFC-236fa), 1,1,2,3,3,3-hexafluoropropane (HFC-236ea), and 1,1,1,4,4,4-hexafluorobutane (HFC-356mffm); of suitable perfluorinated hydrocarbons include compounds such as perfluoropentane or perfluorohexane; and of suitable hydrocarbons include compounds such as various isomers of butane, pentane, cyclopentane, hexane, or mixtures of thereof. Water and carbon dioxide are more preferred blowing agents, with water being most preferred.

Suitable surfactants to be used in accordance with the present invention include, for example, any of the known surfactants which are suitable for production of polyurethane foams. These include, for example, but are not limited to silicone-type surfactants, fluorine-type surfactants, organic surfactants, etc. Organo-silicone copolymer surfactants are widely used in the production of polyurethane foams with polysiloxane-polyoxyalkylene copolymers representing a preferred class. Some examples of suitable surfactants include those compounds commercially available from Degussa - Goldschmidt, General Electric, Air Products, etc. such as those sold as NIAX Silicones L-620, L-5614, L-627, L-6164, L-3858, L-629, L-635, U-2000, etc., and TEGOSTAB Silicones B-8002, B-2370, B-8229, B-8715F, B-8715LF, B-8719LF, etc., and DABCO DC5043, DC5160, DC5169, DC5164, etc.

In addition, it is possible to include additives in the foams of the invention such as, for example, release agents, pigments, cell regulators, flame retarding agents, plasticizers, dyes, antistatic agents, antimicrobials, cross-linking agents, antioxidants, UV stabilizers, mineral oils, fillers and reinforcing agents such as glass in the form of fibers or flakes or carbon fibers.

The foams of the present invention are prepared by mixing together the stable liquid, allophanate-modified, partially trimerized diphenylmethane diisocyanates of the invention with a suitable isocyanate reactive component, one or more blowing agents, one or more catalysts, one or more surfactants, and optionally various other additives known to those in the art. After mixing, the foaming mixture may be deposited into an open container or continuously onto a moving conveyor and be allowed to rise freely (free-rise process). The open container or conveyor may be enclosed in a chamber to provide for foam rise under vacuum or with increased pressure (Variable Pressure Foaming Process). The foaming mixture may also be deposited into a mold that is then closed thus forcing the foam to take on the shape of the mold (molded process).

The reactants are used in quantities such that the Isocyanate Index is from about 100 to 130, preferably from about 100 to about 115. By "isocyanate index" is meant the quotient of the number of isocyanate groups divided by the number of isocyanate-reactive groups, multiplied by 100.

The following examples further illustrate details for the preparation and use of the compositions of this invention. The invention, which is set forth in the foregoing disclosure, is not to be limited either in spirit or scope by these examples. Those skilled in the art will readily understand that known variations of the conditions and processes of the following preparative procedures can be used to prepare these compositions. Unless otherwise noted, all temperatures are degrees Celsius and all parts and percentages are parts by weight and percentages by weight, respectively.

EXAMPLES

The following components were used in the examples:

| | |
|---|---|
| Isocyanate 1:diphenylmethane | diisocyanate having an NCO group content of about 33.5% by wt., and comprising about 98.5% of the 4,4'-isomer of MDI and about 1.5% by weight of the 2,4'-isomer of MDI |
| Isocyanate | 2:diphenylmethane diisocyanate having an NCO group content of about 33.5% by wt. and comprising about 42% by weight of the 4,4'-isomer of MDI, about 57% by weight of the 2,4'-isomer of MDI and less than 1 % by weight of the 2,2'-isomer of MDI |
| Allophanate | A: an allophanate-modified diphenylmethane diisocyanate having an NCO group content of 26% by weight and comprising the reaction product of Isocyanate 1 with isobutanol at an equivalent to equivalent ratio of 1.000 :0.09 in the presence of ZnAcAc. |

Allophanate A was prepared in accordance with the following procedure:

284.8 g (2.35 eq) Isocyanate 1 were added to a dry 1 liter three-neck flask fitted with agitator, thermocouple, and nitrogen inlet. This was heated to 80°C and 15.32 g (0.20 eq) isobutanol were added. Heating continued to 90°C and 0.0361 g of ZnAcAc were added. The allophanate reaction was allowed to proceed for 1.5 hours to give a product with an NCO group content of 26% by weight.

| | |
|---|---|
| Allophanate B: | an allophanate-modified diphenylmethane diisocyanate having an NCO group content of 23% by wt. and comprising the reaction product of Isocyanate 1 with Isobutanol at an equivalent to equivalent ratio of about 1.000 : 0.13 in the presence of ZnAcAc. |
| Allophanate C: | an allophanate-modified diphenylmethane diisocyanate having an NCO group content of 29% by wt. and comprising the reaction product of Isocyanate 1 with isobutanol at an equivalent to equivalent ratio of 1.000 : 0.06 in the presence of ZnAcAc. |

Trimer Allophanate A was prepared in accordance with the following procedure:

300 g Allophanate A were added to a dry 1 liter three-neck flask fitted with agitator, thermocouple, and nitrogen inlet. To this, 200 g Isocyanate 2 were added. The reaction temperature was controlled at 85°C. A trimerization catalyst, i.e: dimethylaminophenol (0.2052 g), was added and the reaction mixture was allowed to trimerize for 1 hour at 85°C Then, 0.1117 g of benzoyl chloride (BzCl) were added to stop the reaction. The NCO content of Trimer Allophanate A was 26% NCO. This product (Trimer Allophanate A) remained a clear liquid at room temperature for more than two weeks.

Examples 1 through 16:

Four 1000g mixtures were made according to the ratios of Allophanate (either Allophanate B or Allophanate C) and Isocyanate 2 as described in the Table headings. For each mixture, the procedure described above for Trimer Allophanate A was used, except that Examples 1-16 used 2,4,6-tris[(N,N-dimethylamino)methyl]phenol as the trimerization catalyst. 50 g aliquot samples were removed from the reaction mixture and treated with 250 ppm benzoyl chloride to yield the final products. Isocyanate content, viscosity and initial appearance of these products were determined. Appearance of the products was also determined after storage at the various times as shown in each Table.

Table 1: Trimerization Product of 90% by wt. of Allophanate C and 10% by weight of Isocyanate 2

| Example | %NCO | Viscosity* | Initial Appearance | Appearance at 24 Hrs. | Appearance at 48 Hrs. | Appearance at 72 Hrs. | Appearance at 7 Days | Appearance at 8 Days |
|---------|-------|-----------|--------------------|-----------------------|-----------------------|-----------------------|----------------------|----------------------|
| 1 | 29.06 | 41.3 | Clear | Clear | Clear | Clear | Hazy | Hazy |
| 2 | 28.69 | 45.2 | Clear | Clear | Clear | Clear | Hazy | Hazy |
| 3 | 27.77 | 180.9 | Clear | Clear | Hazy | Hazy | Hazy | Turbid |
| 4 | 26.18 | 300.8 | Clear | Clear | Hazy | Hazy | Hazy | Turbid |
| 5 | 22.95 | >330,000 | Turbid | Turbid | Turbid | Turbid | Turbid | Turbid |
| * Viscosity measured in mPa·sec at 25°C | | | | | | | | |

Table 2: : Trimerization Product of 75% by wt. of Allophanate C and 25% by weight of Isocyanate 2

| Example | %NCO | Viscosity* | Initial Appearance | Appearance at 24 Hrs. | Appearance at 48 Hrs. | Appearance at 6 Days | Appearance at 7 Days |
|---------|------|-----------|-------------------|----------------------|----------------------|---------------------|---------------------|
| 6 | 29.14 | 44.2 | Clear | Clear | Clear | Hazy | Hazy |
| 7 | 28.36 | 62.9 | Clear | Clear | Slightly Hazy | Hazy | Turbid |
| 8 | 26.06 | 516.1 | Clear | Clear | Hazy | Hazy | Turbid |
| 9 | 23.22 | 130,000 | Hazy | Hazy | Hazy | Hazy | Turbid |
| * Viscosity measured in mPa·sec at 25°C | | | | | | | |

Table 3: Trimerization Product of 90% by wt. of Allophanate B and 10% by weight of Isocyanate 2

| Example | %NCO | Viscosity* | Initial Appearance | Appearance at 24 Hrs. | Appearance at 48 Hrs. | Appearance at 72 Hrs. | Appearance at 7 Days |
|---------|------|-----------|-------------------|----------------------|----------------------|----------------------|---------------------|
| 10 | 23.81 | 355.8 | Clear | Clear | Clear | Clear | Clear |
| 11 | 23.46 | 551.5 | Clear | Clear | Clear | Clear | Clear |
| 12 | 23.1 | 753.0 | Clear | Clear | Clear | Clear | Clear |
| 13 | 20.16 | 47643 | Clear | Clear | Clear | Clear | Clear |
| * Viscosity measured in mPa·sec at 25°C | | | | | | | |

Table 4: Trimerization Product of 75% by wt. of Allophanate B and 25% by weight of Isocyanate 2

| Example | %NCO | Viscosity* | Initial Appearance | Appearance at 24 Hrs. | Appearance at 5 days |
|---------|------|-----------|-------------------|----------------------|---------------------|
| 14 | 24.15 | 375.8 | Clear | Clear | Clear |
| 15 | 22.74 | 2149 | Clear | Clear | Clear |
| 16 | 9.96 | 251,000 | Clear | Clear | Clear |
| * Viscosity measured in mPa·sec at 25°C | | | | | |

The two-day storage data of Tables 1 through 4 were treated to regression analysis using a quadratic model. The appearance of the two-day storage data was coded as follows:

Clear                      = 1.0
Slightly Hazy         = 0.8
Hazy                      = 0.5
Turbid or Opaque   = 0.0

The regression was statistically significant with >99.9% confidence. The regression plots are given for high and low levels of allophanate content in Figures 1 and 2.

Allophanate D:    a commercially available allophanate-modified diphenylmethane diisocyanate having an NCO group content of 26% by weight and comprising the reaction product of Isocyanate 1 with isobutanol at an equivalent to equivalent ratio of 1.000 :0.09 in the presence of ZnAcAc. In the commercial preparation of this allophanate-modified product, benzoyl chloride is typically added as a catalyst stopper in an amount of about 38%. This product is commercially available from Bayer MaterialScience LLC.

Example 17:

The allophanate trimer of this example was prepared in a 5L reactor fitted with an agitator, thermocouple, and nitrogen inlet. To the reactor was added 2400 g of Allophanate D, a commercially available allophanate product and 1600 g of Isocyanate 2. The blend was heated to 65 °C under flowing nitrogen. 400 ppm of Ancamine 1110 (commercially available from Air Products), a trimer catalyst, was added to the blend which was then heated to 80 °C. The trimerization reaction was allowed to progress for approximately 90 minutes. The reaction was stopped by adding 150 ppm of benzoyl chloride at a final %NCO of 26.2. The product was a clear liquid trimer allophanate at room temperature. This product remained clear for at least 2 months.

Although the invention has been described in detail in the foregoing for the purpose of illustration, it is to be understood that such detail is solely for that purpose and that variations can be made therein by those skilled in the art without departing from the spirit and scope of the invention except as it may be limited by the claims.

## Claims

1. A stable liquid, allophanate-modified, partially trimerized diphenylmethane diisocyanate having an NCO group content of 10% to 30%, and comprising the trimerization product of:

   (A) from 75 to 90% by weight, based on 100% by weight of (A) and (B), of an allophanate-modified diphenylmethane diisocyanate component having an NCO group content of 23% to 29%, and which comprises the reaction product of:

   (1) diphenylmethane diisocyanate which comprises from 85% to 100% by weight of the 4,4'-isomer, from 0 to 9% by weight of the 2,4'-isomer and from 0 to 6% by weight of the 2,2'-isomer, with the sum of the %'s by weight of the 4,4'-isomer, the 2,4'-isomer and the 2,2'-isomer totaling 100% by weight of the diphenylmethane diisocyanate component;
   with
   (2) an organic compound having from 1 to 36 carbon atoms and containing at least one hydroxyl group;
   in the presence of
   (3) an allophanate catalyst;

   and
   (B) from 10 to 25% by weight, based on 100% by weight of (A) and (B), of a diphenylmethane diisocyanate component having an NCO group content of about 33 to about 34% by weight, and comprising from 31 % to 55% by weight of the 4,4'-isomer, from 45% to 65% by weight of the 2,4'-isomer and from 0 to 4% by weight of the 2,2'-isomer, with the sum of the %'s by weight of the 4,4'-isomer, the 2,4'-isomer and the 2,2'-isomer totaling 100% by weight of the diphenylmethane diisocyanate component;
   in the presence of
   (C) at least one trimerization catalyst; with the proviso that the clarity of the allophanate-modified, partially trimerized diphenylmethane diisocyanate product after 2 days is $\geq 0.8$, and in which:

   $$0.8 \leq ((17.19 \times WF_A) + (0.94 \times WF_B) - (0.54 \times WF_A \times \%NCO_P) - (0.70 \times WF_A \times \%NCO_A) + (0.02 \times WF_A \times \%NCO_P \times \%NCO_A))$$

   wherein:

   $WF_A$: represents the weight fraction of the allophanate-modified diphenylmethane diisocyanate component (A);
   $WF_B$: represents the weight fraction of the diphenylmethane diisocyanate component (B);
   $NCO_A$: represents the NCO group content of the allophanate-modified diphenylmethane diisocyanate component (A);
   and
   $NCO_P$: represents the NCO group content of the allophanate-modified, partially trimerized diphenylmethane diisocyanate product which ranges from 10% to 30%.

2. The stable liquid, allophanate-modified, partially trimerized diphenylmethane diisocyanate of Claim 1, wherein (A) (2) said organic compound is selected from the group consisting of aromatic alcohols having from 6 to 18 carbon atoms and aliphatic alcohols having from 1 to 36 carbon atoms.

3. The stable liquid, allophanate-modified, partially trimerized diphenylmethane diisocyanate of Claim 1, wherein the NCO group content is from 15 to 30% by weight.

4. The stable liquid, allophanate-modified, partially trimerized diphenylmethane diisocyanate of Claim 1, which comprises the trimerization product of:

(A) from 78 to 88% by weight, based on 100% by weight of (A) and (B), of an allophanate-modified diphenylmethane diisocyanate component having an NCO group content of 25 to 29% by weight,
and
(B) from 12 to 22% by weight, based on 100% by weight of (A) and (B), of a diphenylmethane diisocyanate component having an NCO group content of about 33 to about 34% by weight, and comprising from about 40 to 50% by weight of the 4,4'-isomer, from about 50 to about 60% by weight of the 2,4'-isomer and less than 2% by weight of the 2,2'-isomer, with the sum of the %'s by weight of the 4,4'-isomer, the 2,4'-isomer and the 2,2'-isomer totaling 100% by weight of the diphenylmethane diisocyanate component.

5. The stable liquid, allophanate-modified, partially trimerized diphenylmethane diisocyanate of Claim 4, wherein (A) said allophanate-modified diphenylmethane diisocyanate component comprises the reaction product of:

(1) diphenylmethane diisocyanate which comprises from about 90 to about 98% by weight of the 4,4'-isomer, from about 0.5 to about 4% by weight of the 2,4'-isomer and from 0 to about 0.5% by weight of the 2,2'-isomer, with the sum of the %'s by weight of the 4,4'-isomer, the 2,4'-isomer and the 2,2'-isomer totaling 100% by weight of the diphenylmethane diisocyanate;
and
(2) an organic compound selected from the group consisting of aromatic alcohols having from 6 to 18 carbon atoms and aliphatic alcohols having from 1 to 36 carbon atoms.

6. The stable liquid, allophanate-modified, partially trimerized diphenylmethane diisocyanate of Claim 1, wherein (A) (2) said organic compound is selected from the group consisting of 2-propanol, isobutyl alcohol and phenol.

7. The stable liquid, allophanate-modified, partially trimerized diphenylmethane diisocyanate of Claim 1, wherein (A) (3) said allophanate catalyst is selected from the group consisting of zinc octoate, tin octoate, zinc-2-ethylhexanoate, tin-2-ethylhexanoate and zinc acetylacetonate.

8. The stable liquid, allophanate-modified, partially trimerized diphenylmethane diisocyanate of Claim 1, wherein (C) said trimerization catalyst is selected from the group consisting of dimethylaminophenol and 2,4,6-bis(dimethylaminomethyl)-phenol.

9. A process for the preparation of a stable liquid, allophanate-modified, partially trimerized diphenylmethane diisocyanate having an NCO group content of 10% to 30% by weight, comprising:

(I) trimerizing a blend comprising:

(A) from 75 to 90% by weight, based on 100% by weight of (A) and (B), of an allophanate-modified diphenylmethane diisocyanate component having an NCO group content of 23% to 29%, and which comprises the reaction product of:

(1) diphenylmethane diisocyanate which comprises from 85% to 100% by weight of the 4,4'-isomer, from 0 to 9% by weight of the 2,4'-isomer and from 0 to 6% by weight of the 2,2'-isomer, with the sum of the %'s by weight of the 4,4'-isomer, the 2,4'-isomer and the 2,2'-isomer totaling 100% by weight of the diphenylmethane diisocyanate component;
with
(2) an organic compound having from 1 to 36 carbon atoms and containing at least one hydroxyl group (preferably an aromatic alcohol having from 6 to 18 carbon atoms, or more preferably an aliphatic alcohol having from 1 to 36 carbon atoms);
in the presence of
(3) an allophanate catalyst;

with

(B) from 10 to 25% by weight, based on 100% by weight of (A) and (B), of a diphenylmethane diisocyanate component having an NCO group content of about 33 to about 34% by weight, and containing from 31% to 55% by weight of the 4,4'-isomer, from 45% to 65% by weight of the 2,4'-isomer and from 0 to 4% by weight of the 2,2'-isomer, with the sum of the %'s by weight of the 4,4'-isomer, the 2,4'-isomer and the 2,2'-isomer totaling 100% by weight of the diphenylmethane diisocyanate component;
in the presence of:
(C) at least one trimerization catalyst;

and
(II) adding a suitable catalyst stopper once the desired NCO group content is attained;
with the proviso that the clarity of the allophanate-modified, partially trimerized diphenylmethane diisocyanate product after 2 days is $\geq 0.8$, and in which:

$$0.8 \leq ((17.19 \times WF_A) + (0.94 \times WF_B) - (0.54 \times WF_A \times \%NCO_P) -$$
$$(0.70 \times WF_A \times \%NCO_A) + (0.02 \times WF_A \times \%NCO_P \times \%NCO_A))$$

wherein:

$WF_A$: represents the weight fraction of the allophanate-modified diphenylmethane diisocyanate component (A);
$WF_B$: represents the weight fraction of the diphenylmethane diisocyanate component (B);
$NCO_A$: represents the NCO group content of the allophanate-modified diphenylmethane diisocyanate component (A);
and
$NCO_P$: represents the NCO group content of the allophanate-modified, partially trimerized diphenylmethane diisocyanate product which ranges from 10% to 30%.

10. The process of Claim 9, wherein (A)(2) said organic compound is selected from the group consisting of aromatic alcohols having from 6 to 18 carbon atoms and aliphatic alcohols having from 1 to 36 carbon atoms.

11. The process of Claim 9, wherein the NCO group content is from 15 to 30% by weight.

12. The process of Claim 9, which comprises the trimerization product of:

(A) from 78 to 88% by weight, based on 100% by weight of (A) and (B), of an allophanate-modified diphenylmethane diisocyanate component having an NCO group content of 25 to 29% by weight,
and
(B) from 12 to 22% by weight, based on 100% by weight of (A) and (B), of a diphenylmethane diisocyanate component having an NCO group content of about 33 to about 34% by weight, and comprising from about 40 to 50% by weight of the 4,4'-isomer, from about 50 to about 60% by weight of the 2,4'-isomer and less than 2% by weight of the 2,2'-isomer, with the sum of the %'s by weight of the 4,4'-isomer, the 2,4'-isomer and the 2,2'-isomer totaling 100% by weight of the diphenylmethane diisocyanate component.

13. The process of Claim 12, wherein (A) said allophanate-modified diphenylmethane diisocyanate component comprises the reaction product of:

(1) diphenylmethane diisocyanate which comprises from about 90 to about 98% by weight of the 4,4'-isomer, from about 0.5 to about 4% by weight of the 2,4'-isomer and from 0 to about 0.5% by weight of the 2,2'-isomer, with the sum of the %'s by weight of the 4,4'-isomer, the 2,4'-isomer and the 2,2'-isomer totaling 100% by weight of the diphenylmethane diisocyanate;
and
(2) an organic compound selected from the group consisting of aromatic alcohols having from 6 to 18 carbon atoms and aliphatic alcohols having from 1 to 36 carbon atoms.

14. The process of Claim 9, wherein (A)(2) said organic compound is selected from the group consisting of 2-propanol, isobutyl alcohol and phenol.

**15.** The process of Claim 9, wherein (A)(3) said allophanate catalyst is selected from the group consisting of zinc octoate, tin octoate, zinc-2-ethylhexanoate, tin-2-ethylhexanoate and zinc acetylacetonate.

**16.** The process of Claim 9, wherein (C) said trimerization catalyst is selected from the group consisting of dimethylaminophenol and 2,4,6-bis(dimethylaminomethyl)-phenol.

**17.** A process for the production of a polyurethane foam comprising reacting

(1) a polyisocyanate component comprising the stable, liquid allophanate-modified partially trimerized diphenylmethane diisocyanate of Claim 1;
with
(2) an isocyanate-reactive component
in the presence of
(3) one or more catalysts;
(4) one or more blowing agents *(preferably water)*;
and
(5) at least one surfactant;
at an Isocyanate Index of 100 to 130.

**18.** The process of Claim 17, in which (2) said isocyanate-reactive component comprises:

(a) from 45% to 75% by weight of at least one aromatic polyester polyol component having a functionality of about 2 and a molecular weight of from about 500 to about 3000 and an OH number of from about 35 to about 2250,
(b) from 5% to 15% by weight of at least one glycol extender having a functionality of 2, a molecular weight of 60 to 300 and an OH number of 370 to 1870;
and
(c) from 20% to 45% by weight of at least one crosslinking agent having a functionality of from about 3 to about 4, a molecular weight of about 60 to about 500 and an OH number of from about 330 to about 3750;
with the %'s by weight of (a), (b) and (c) totaling 100% by weight of the isocyanate-reactive component (2).

**19.** The polyurethane foam produced by the process of Claim 17.

**European Patent Office**

**EUROPEAN SEARCH REPORT**

Application Number

EP 08 00 4049

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/047362 A (BAYER MATERIALSCIENCE LLC [US]; SLACK WILLIAM E [US]) 26 May 2005 (2005-05-26) | 1-19 | INV. C08G18/76 C08G18/78 |
| Y | * claim 1; example 20 * | 1-19 | C08G18/79 |
| | ----- | | |
| Y | WO 2006/039298 A (BAYER MATERIALSCIENCE LLC [US]; KAUSIVA BRYAN D [US]; SLACK WILLIAM E) 13 April 2006 (2006-04-13) * page 18, line 8 - line 19; table II * * page 6, line 28 - line 32 * | 1-19 | |
| | ----- | | |
| A | EP 0 031 207 A (ICI PLC [GB]) 1 July 1981 (1981-07-01) * page 4, line 1 - line 10 * * page 5, line 8 - line 20 * | 1-19 | |
| | ----- | | |

**TECHNICAL FIELDS SEARCHED (IPC)**

C08G

The present search report has been drawn up for all claims

| Place of search | Date of completion of the search | Examiner |
|---|---|---|
| Munich | 23 July 2008 | Scheuer, Sylvie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 00 4049

This annex lists the patent family members relating to the patent documents cited in  the above-mentioned European search report.
The members are as contained in the European Patent Office EDP file on
The European Patent Office is in no way liable for these particulars which are merely given for the purpose of information.

23-07-2008

| Patent document cited in search report | | Publication date | Patent family member(s) | | Publication date |
|---|---|---|---|---|---|
| WO 2005047362 | A | 26-05-2005 | BR | PI0416561 A | 23-01-2007 |
| | | | CA | 2545147 A1 | 26-05-2005 |
| | | | CN | 1878816 A | 13-12-2006 |
| | | | EP | 1685177 A1 | 02-08-2006 |
| | | | JP | 2007510802 T | 26-04-2007 |
| | | | KR | 20060110296 A | 24-10-2006 |
| | | | US | 2007129526 A1 | 07-06-2007 |
| | | | US | 2005101754 A1 | 12-05-2005 |
| WO 2006039298 | A | 13-04-2006 | AU | 2005292105 A1 | 13-04-2006 |
| | | | CA | 2521571 A1 | 01-04-2006 |
| | | | CN | 101031601 A | 05-09-2007 |
| | | | EP | 1797130 A2 | 20-06-2007 |
| | | | JP | 2008514792 T | 08-05-2008 |
| | | | KR | 20070073843 A | 10-07-2007 |
| | | | MX | PA05010514 A | 11-07-2006 |
| | | | US | 2006073321 A1 | 06-04-2006 |
| EP 0031207 | A | 01-07-1981 | DE | 3069424 D1 | 15-11-1984 |
| | | | JP | 1607571 C | 13-06-1991 |
| | | | JP | 2034940 B | 07-08-1990 |
| | | | JP | 56092855 A | 27-07-1981 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4743627 A
- US 4382125 A
- US 4284730 A
- US 5124370 A
- US 5905151 A
- US 5955609 A
- US 6127308 A
- US 6028158 A
- US 6063891 A
- US 4255659 A
- US 4518761 A
- US 4772639 A
- US 5798431 A
- US 5102918 A
- US 4326043 A
- US 4359541 A
- US 4359550 A

- GB 1337659 A
- US 5319053 A
- US 5319054 A
- US 5663272 A
- US 5783652 A
- US 6482913 B
- US 6887399 B
- US 6991746 B
- US 20050101754 A1
- US 20060073321 A1
- US 5440003 A
- US 4604418 A
- US 4379905 A
- US 4487928 A
- US 3954684 A
- US 4632785 A
- US 4540781 A